# NEUE EUROPÄISCHE PATENTSCHRIFT

(11) **EP 1 861 455 B2**
(45) Veröffentlichungstag und Bekanntmachung des Hinweises auf die Entscheidung über den Einspruch: **29.07.2015**
(45) Hinweis auf die Patenterteilung: 08.10.2008
(21) Anmeldenummer: 06723433.6
(22) Anmeldetag: 15.03.2006
(51) Int. Cl.: C08J 11/00, C08J 11/06, C08L 67/02

(54) **VERFAHREN UND VORRICHTUNG ZUR DEKONTAMINATION VON KUNSTSTOFFFLAKES**
METHOD AND DEVICE FOR THE DECONTAMINATION OF PLASTIC FLAKES
PROCEDE ET DISPOSITIF POUR DECONTAMINER DES PAILLETTES DE PLASTIQUE

(30) Priorität: 24.03.2005 DE 102005013701
(43) Veröffentlichungstag der Anmeldung: 05.12.2007
(73) Patentinhaber: Krones AG, 93073 Neutraubling (DE)
(72) Erfinder: FRIEDLAENDER, Thomas, Dr., 93049 Regensburg (DE); RIECKMANN, Thomas, Dr., 34260 Kaufungen (DE); MARX, Frank, 53332 Bornheim (DE)
(74) Vertreter: Grünecker Patent- und Rechtsanwälte PartG mbB
(86) Internationale Anmeldenummer: PCT/EP2006/002356
(87) Internationale Veröffentlichungsnummer: WO 2006/099976

(56) Entgegenhaltungen:
- WO-A-2005/037513
- WO-A-2005/121230
- WO-A1-01/21372
- WO-A1-01/21373
- WO-A1-03/037588
- WO-A1-2004/029130
- CA-A- 2 284 965
- DE-A1- 10 324 098
- DE-A1- 19 953 659
- US-A- 4 370 302
- US-A1- 2001 004 645

## Beschreibung

Die Erfindung betrifft ein Verfahren zur Aufbereitung von zu Flakes zerkleinerten kontaminierten Kunststoffen nach dem Oberbegriff des Anspruchs 1.

Gebrauchsgegenstände aus Kunststoff wie z. B. Getränkeflaschen aus PET, Haushaltsgegenstände wie Schüsseln o. dgl. oder Gehäuseteile von Elektronikgeräten erfreuen sich immer größerer Beliebtheit. Die weltweite Verbreitung von Kunststoffen in den verschiedensten Bereichen hat dabei ein so hohes Ausmaß angenommen, sodass die Rohstoffe inzwischen knapper und somit teuerer werden. Das Recycling von Kunststoffen ist also nicht nur aus Umweltschutzgründen wichtig, sondern wird auch aus wirtschaftlicher Sicht immer interessanter.

Bei steigenden Rohstoffpreisen werden vor allem auch Recyclingverfahren interessant, die es ermöglichen, die Qualität des Ursprungsmaterials bezüglich seiner Bearbeitbarkeit nach dem Recycling beizubehalten bzw. zu erhöhen.

Ursprünglich ging man davon aus, dass man gesammeltes PET-Material (R-PET) aufgrund des Qualitätsverlustes nach dem Recycling und verschiedenen Kontaminationen nicht mehr der gleichen Verwendung (z. B. Getränkeflaschen) zuführen könne. Seit kurzer zeit weiß man, dass dies möglich ist. Es gibt Verfahren, die Kunststoffe auf einem qualitativ hohen Niveau zu recyceln, um sie der gleichen Verwendung (z. B. Lebensmittelbereich) wieder zuführen zu können.

Nach einem Verfahren gemäß DE 199 53 659 A1 werden postconsumer PET-Flaschen (R-PET) zerkleinert und zunächst in üblicher Weise gewaschen und oberflächlich getrocknet. Anschließend gelangen die Flakes in einen Kristallisator, in dem sie von einem 180° C heißen Gasstrom erhitzt und kristallisiert werden. Danach gelangen die Flakes in einen Schachtreaktor, wo sie während mindestens zwei Stunden im Gegenstrom zu einem N₂-Gasstrom (Temperatur ca. 220° C) kontinuierlich nachkondensiert werden. Anschließend werden die Flakes in diesem kontinuierlich ablaufenden Prozess wieder gekühlt. Ein Nachteil dieses Verfahrens besteht darin, dass die Erwärmung der PET-Flakes in den jeweiligen Reaktoren also im Kristallisator bzw. Schachtreaktor erfolgt, weshalb relativ viel Energie zur Erwärmung eingesetzt werden muss. Außerdem wird die Wärmeübertragung über (Inert)gas durchgeführt, was zu einem komplizierteren Aufbereitungssystem für das Wärmeübertragungsmedium führt. Ein weiterer Nachteil des Verfahrens ist, dass die Nachkondensation der Flakes kontinuierlich erfolgt. Der gewünschte IV-Wert lässt sich bei einer kontinuierlichen Prozessführung nicht genau einstellen, da nur eine bestimmte Erhöhung aufgrund der Prozessparameter (z.B. Temperatur und Zeit) realisierbar ist. Die Tatsache, dass der reale IV-Wert der zu behandelnden Flakes unterschiedlich sein kann - zum Beispiel haben einige Flakes eine intrinsische Viskosität (IV) von 0,70 dl/g, andere haben bereits einen Wert von 0,76 dl/g - wird bei dieser Verfahrensweise nicht berücksichtigt, sodass alle Flakes ohne Berücksichtigung ihres ursprünglichen IV-Wertes in gleicher Weise (selbe Zeit, selbe Prozessbedingungen) in ihrer intrinsischen Viskosität erhöht werden.

Es ist deshalb Aufgabe der vorliegenden Erfindung, ein Verfahren zum Recyceln von Kunststoffen bereitzustellen, dessen bzw. deren Energieverbrauch stark reduziert ist und bei dem bzw. der der IV-Wert der Kunststoffe gezielt eingestellt werden kann.

Erfindungsgemäß wird die Aufgabe bezüglich des Verfahrens gemäß dem Kennzeichen des Anspruchs 1 gelöst.

Der Erfindung liegt die Erkenntnis zugrunde, dass die Kontaminanten nicht gleichmäßig über das R-PET verteilt sind und zudem vorrangig auf der Oberfläche bzw. oberflächennahen Bereichen vorliegen. Es ist daher eine Dekontamination und/oder SSP-Behandlung ausreichend, um R-PET lebensmitteltauglich zu reinigen und erneut als Verpackung für Lebensmittel, z. B. für Getränkeflaschen zu verwenden. Außerdem liegt der Erfindung die überraschende Erkenntnis zugrunde, dass R-PET im Gegensatz zu neuem (virgin) PET im warmen Zustand nicht klebt, womit eine störungsfreie prozesstechnische Verarbeitung (z.B. Erwärmung des R-PET in einer Heizeinrichtung) erst möglich wird. Dies liegt daran, dass sich die R-PET-Flakes überraschender Weise bei der Erwärmung zusammenziehen und dadurch ihre Dicke bzw. ihre kennzeichnende Länge erhöht wird. Gleichzeitig entsteht eine strukturierte Oberfläche, die die Kontaktfläche der Flakes untereinander reduziert.

Bezüglich der Durchführbarkeit des Verfahrens spielt die Größe der Flakes grundsätzlich keine Rolle. Mit steigender Partikelgröße ändert sich lediglich die Verweildauer in den Reaktoren. Je größer die durchschnittliche Partikelgröße ist, desto länger ist auch die Verweildauer im Reaktor. Aus diesem Grund ist es also auch denkbar, dass Kunststoffgegenstände vor ihrer Behandlung nicht bzw. nur wenig zerkleinert werden. So sind unter Flakes alle Gegenstände zu verstehen, die in ihrer Form und Größe nicht mehr ihrer ursprünglichen Verwendungsgestalt entsprechen. In aller Regel haben die Flakes aber eine durchschnittliche Partikelgröße von einigen Millimetern bis einigen Zentimetern.

Die Vorbereitung der Flakes vor der Aufbereitung, also die Reinigung und die Zerkleinerung, erfolgt meist in größeren Sammelzentren, es ist jedoch auch denkbar, dass die Zerkleinerung und (Grob)reinigung der Flakes erst direkt vor Ort vor der Aufbereitung stattfindet.

Unter Aufbereitung versteht man hier nicht nur die oberflächliche Reinigung bzw. Dekontamination der Flakes, sondern auch die Verbesserung bzw. Wiederherstellung der physikalischen und der chemischen Eigenschaften der Flakes, um insbesondere für die Verpackung von z.B. flüssigen Lebensmitteln im direkten Kontakt mit dem Kunststoff eine erneute hochwertige Nutzung (wie z. B. Streckblasen von Getränkeflaschen) sicherzustellen.

Da das Verfahren sowohl in großtechnischem Maßstab als auch in kleinen Anlagen umsetzbar ist, versteht man unter Reaktoren sowohl große Kessel, Behälter oder dgl., als auch kleinere Behälter, die gegenüber der Umgebung im Wesentlichen abgeschlossen werden können.

Die Erwärmung der zu behandelnden Flakes auf Prozesstemperatur erfolgt erfindungsgemäß im Wesentlichen in einer Behandlungseinheit, die sich außerhalb des Reaktors befindet. Bei dieser Erwärmung kann es sich sowohl um eine kontinuierliche als auch um eine chargenweise Erwärmung der Flakes handeln. Im Reaktor selbst werden die Flakes kaum bzw. nicht mehr erwärmt, sondern nur noch auf Prozesstemperatur gehalten bzw. Wärmeverluste ausgeglichen. Es handelt sich also hierbei um eine im Wesentlichen adiabate Prozessführung.

Unter SSP versteht man den Prozess der Molekülkettenverlängerung der Kunststoffe in fester Phase (solid state polycondensation). Der SSP-Prozess ist ein Standardprozess, den jeder Fachmann auf dem Gebiet der PET-Kunststofftechnik kennt. Mit SSP-Behandlung kann hier auch eine negative Molekülkettenverlängerung, also Depolymerisation oder eine Behandlung, bei der die Molekülketten im Durchschnitt im Wesentlichen gleich bleiben, gemeint sein. Egal ob die Molekülketten verkürzt werden, verlängert werden oder gleich bleiben, findet während des SSP-Prozesses aufgrund der Prozessbedingungen (u.a. Temperatur, Zeit) auch eine weitere Dekontamination der Flakes statt.

Erfindungsgemäß ist eine Prozessführung, bei der sowohl die Dekontamination als auch die SSP-Behandlung in jeweils einem eigenen Reaktor stattfindet. Die beiden Reaktoren sind somit zeitgleich bezüglich ihrer Reaktionsbedingungen (Druck, Temperatur, Gasart) unabhängig voneinander einstellbar.

Vorzugsweise herrschen in beiden Reaktoren adiabate Bedingungen vor. Es ist denkbar, dass die geringen Wärmeverluste der Flakes in den Reaktoren durch eine Reaktorbeheizung ausgeglichen werden. Dabei ist eine wie auch immer geartete Beheizung möglich (z.B. Wasser, Dampf, elektrisch, Wärmeträgeröl wie Thermalöl).

Gemäß einer besonders bevorzugten Ausführungsform wird das aufzubereitende Material in den Rektoren nicht in Bewegung gebracht, weder mechanisch, noch pneumatisch noch auf eine andere Art und Weise. Dadurch können sich die Flakes während des Prozesses der Dekontamination bzw. der SSP-Behandlung auf Grund von Reibung aneinander bzw. Interaktion mit den Reaktorwänden nicht statisch aufladen bzw. es bilden sich keine Abrasionsprodukte, die den weiteren Prozessablauf erschweren, verkomplizieren oder gar unmöglich machen würden.

Zur Abfuhr der Kontaminanten werden die Flakes während des Schrittes der Dekontamination von heißem Gas umströmt. Bei dem heißen Gas handelt es sich vorzugsweise um Luft, um eine möglichst einfache Prozessführung zu gewährleisten. Im Dekontaminationsreaktor herrscht dabei eine Temperatur zwischen 20 und 200° C, bevorzugt jedoch zwischen 100° C und 180° C am besten zwischen 150° C und 170° C. Die Funktionsfähigkeit des Prozesses ist bei allen angegebenen Temperaturen gewährleistet, es ändert sich lediglich die Verweildauer im Reaktor. Je höher die Temperatur im Dekontaminationsreaktor ist, desto geringer ist die Verweildauer. Der optimale Arbeitspunkt des Dekontaminationsreaktors bezüglich der Temperatur liegt deshalb zwischen 150° C und 170° C, da hier einerseits die Temperatur relativ hoch und somit die Verweildauer im Rektor relativ kurz ist, andererseits als Gas zur Abfuhr der Kontaminanten Luft verwendet werden kann. Bei Temperaturen über ca. 180° C kann Luft auf Grund verschiedener Reaktionen, die dann mit dem Material eingegangen werden, nicht mehr als Heißgas verwendet werden. In diesem Fall müsste auf z. B. Stickstoff zurückgegriffen werden. Der Prozess der Dekontamination kann auch unterhalb einer Temperatur von 20° C durchgeführt werden, allerdings wird die Verweildauer im Reaktor dann sehr groß. Das Gas hat eine Temperatur, die im Wesentlichen der des Dekontaminationsprozesses entspricht. Dadurch können Wärmeverluste der Flakes ausgeglichen werden.

Bezüglich der Erwärmung der Flakes auf Prozesstemperatur gibt es die Möglichkeit der chargenweisen und der kontinuierlichen Erwärmung. Vorzugsweise wird die Erwärmung kontinuierlich möglichst nah am Dekontaminationsreaktor durchgeführt. Je näher das Heizmodul am Dekontaminationsreaktor ist, desto geringer ist die Bildung von Abrasionsprodukten beim Transport. Je besser der Wärmeübergang vom Heizmodul auf die Flakes, desto Energiesparender kann der Erwärmungsprozess betrieben werden. Zur kontinuierlichen Erwärmung der Flakes vor der Dekontamination wird vorzugsweise eine Heizschnecke oder ein Vibrationswendelförderer verwendet. Es sind auch andere Heizmodule denkbar, jedoch bieten die Heizschnecke und der Vibrationswendelförderer den Vorteil eines guten Wärmeübergangs auf die Flakes. Vorzugsweise wird als Wärmeträgermedium in der Heizschnecke Thermalöl verwendet, wobei z.B. auch eine Beheizung mit Dampf, mit Heißwasser oder elektrisch vorgenommen werden kann.

In einer bevorzugten Ausführungsform wird der SSP-Prozess unter Unterdruckbedingungen durchgeführt. Zwar ist es auch möglich den Prozess unter Atmosphären- bzw. Überdruckbedingungen stattfinden zu lassen, jedoch bietet das Vakuum den Vorteil, dass der Stoffübergang flüchtiger Verbindungen von den Flakes in die Gasphase verbessert wird und so sowohl die Kontaminanten besser abzuführen sind als auch die Bedingungen zur Molekülkettenverlängerung vorteilhafter sind. Das Vakuum, das für den SSP-Prozess verwendet wird, sollte optimalerweise einen Gasdruck von höchstens 100 Millibar, vorzugsweise zwischen 10 und 0,1 Millibar aufweisen. Wird der SSP-Prozess bei Atmosphärenbedingungen oder Überdruckbedingungen durchgeführt, so ist für eine ausreichende Umströmung der Partikeln zu sorgen, um den Stoffübergang zu verbessern, bzw. die Kontaminanten besser abführen zu können.

Erfindungsgemäß herrschen im SSP-Reaktor zur Einstellung des IV-Wertes höhere Temperaturen als im Dekontaminationsreaktor vor. Die Temperatur beträgt dabei vorzugsweise höchstens den Wert der Schmelztemperatur der zu behandelnden Flakes. Vorzugsweise liegt die Temperatur dabei zwischen 150° C und 250° C besonders bevorzugt zwischen 170° C und 210° C.

Die Erhöhung der Prozesstemperatur von der des Dekontaminationsreaktors auf die des SSP-Reaktors erfolgt in einem separaten Wärmeüberträger, der sich zwischen den beiden Reaktoren befindet. Vorzugsweise handelt es sich dabei wiederum um einen Wärmetauscher bzw. eine Heizeinrichtung, der/die als Wärmeübertragungsmedium Thermalöl verwendet. Der Wärmeüberträger ist gemäß einer besonders bevorzugten Weiterbildung eine Heizschnecke oder ein Vibrationswendelförderer. Die Verwendung eines Heißluftförderers, eines Infrarottunnels oder dergleichen ist auch vorstellbar.

Besondere Vorteile bei der Durchführung des SSP-Prozesses werden dadurch erzielt, dass er unter Inertgasatmosphäre durchgeführt wird. Bei der Verwendung von Stickstoff oder Kohlendioxid als Schutzgas kann der Prozess besonders gut durchgeführt werden. Weitere Vorteile bietet der Einsatz eines Gasstroms, der Abbauprodukte, Kontaminanten, Feinstpartikel oder dgl. aus dem Reaktor ausführt. Vorzugsweise handelt es sich bei dem Gas um das Atmosphärengas, in dem der SSP-Prozess stattfindet. Der Gasstrom ist vorzugsweise auch hier nur so stark, dass die Flakes nicht bewegt werden. Durch den Gasstrom soll lediglich die Abfuhr der Abbauprodukte gewährleistet werden.

Um den Prozess bezüglich der Einstellung des IV-Wertes der Flakes zu optimieren, wird der aktuelle Wert der intrinsischen Viskosität während des Aufbereitungsprozesses mindestens einmal bestimmt. Besonders bevorzugt ist jedoch die Vorgehensweise, bei der IV-Wert mindestens zweimal gemessen wird, wobei die Ergebnisse der Messungen den weiteren Prozessablauf wie z. B. die Verweildauer oder den Temperaturverlauf bestimmen.

Besonders bevorzugt ist dabei ein Verfahren, bei dem der IV-Wert räumlich und / oder zeitlich vor dem Eintritt des aufzubereitenden Materials in den SSP-Reaktor bestimmt wird. Anhand dieses Wertes kann bei fester Temperatur die Verweildauer im SSP-Reaktor bestimmt werden. Nach der Hälfte der berechneten Verweildauer wird abermals eine Messung des IV-Wertes durchgeführt und anhand dieses Ergebnisses die restliche Verweilzeit kontrolliert bzw. ggf. korrigiert. Die Messung des IV-Wertes der Flakes kann sowohl im Reaktor als auch außerhalb desselben durchgeführt werden, wobei die Probennahme mittels einer Flakeprobenentnahmevorrichtung erfolgt.

Es ist aber auch ein Verfahren denkbar, bei dem eine Messung des IV-Wertes durchgeführt wird und Anhand dessen Ergebnisses die Temperatur des SSP-Prozesses bei fester Verweildauer festgelegt wird. Nach der Hälfte der festen Verweildauer kann abermals eine Messung des IV-Wertes durchgeführt werden, wobei Anhand des Ergebnisses die Temperatur für die restliche Laufzeit des Prozesses kontrolliert bzw. korrigiert wird. Auch bei dieser Verfahrensvariante kann die Messung des IV-Wertes analog der obigen Ausführung sowohl innen als auch außen durchgeführt werden.

Ergibt die Messung der intrinsischen Viskosität vor der SSP-Behandlung bereits das gewünschte Ergebnis, so wird der Prozess so eingestellt, dass sich der IV-Wert nicht mehr ändert. Dies kann zum Beispiel dadurch erfolgen, dass der Feuchtigkeitsgehalt der Flakes entsprechend eingestellt wird, wodurch sich die Molekülkettenverkürzung durch Hydrolyse und die Molekülkettenverlängerung durch Polykondensation im Mittel ausgleichen. Dadurch bleibt der Wert der intrinsischen Viskosität in Summe gleich.

Ergibt die Messung der intrinsischen Viskosität vor der SSP-Behandlung einen zu hohen IV-Wert, so wird eine "negative" SSP-Behandlung, also eine gezielte Depolymerisation, vorgenommen, indem der Feuchtigkeitsgehalt der Flakes entsprechend eingestellt wird, sodass die Molekülkettenverkürzung durch Hydrolyse gegenüber der Molekülkettenverlängerung durch Polykondensation überwiegt. Dadurch entsteht ein niedrigerer IV-Wert.

Ist die Einstellung des IV-Wertes im SSP-Reaktor abgeschlossen, so kann eine Abkühlung der Flakes auf eine Temperatur zwischen 50° C und 100° C bzw. in einer bevorzugten Ausführungsform von weniger als 70°C vorgenommen werden. Die Abkühlung erfolgt vorzugsweise in einer Kühlschnecke, einem Vibrationswendelförderer oder in einem Wirbelschichtreaktor, wobei auch andere Kühlvorrichtungen vorgesehen sein können. Erfolgt die Abkühlung durch einen kalten Gasstrom, so wird vorzugsweise Umgebungsluft verwendet, wodurch ein einfacher Kühlprozess gewährleistet ist. Bei der Kühlung durch Umgebungsluft ist darauf zu achten, dass der gesamte Kühlvorgang nicht zu lange dauert und dass insbesondere die Abkühlung im Temperaturintervall von der Reaktionstemperatur auf 150°C sehr schnell erfolgt, bevorzugt in einer Zeit zwischen einer und drei Minuten. Ist eine Abkühlung in dieser Geschwindigkeit nicht möglich, so muss bei einem gasförmigen Kühlmedium ein Schutzgas, vorzugsweise Stickstoff oder Kohlendioxid, verwendet werden. Die Verwendung eines anderen Kühlmediums wie zum Beispiel einer Kühlflüssigkeit ist genauso möglich.

Die Vorrichtung zur Durchführung des Verfahrens weist mindestens zwei Reaktoren auf, von denen einer ein Dekontaminationsreaktor und einer ein SSP-Reaktor zur Einstellung des IV-Wertes ist. In den Reaktoren können beliebige Kunststoffflakes aufbereitet werden, vorzugsweise handelt es sich dabei um PET-Flakes.

Dem Dekontaminationsreaktor vorgeschaltet ist eine Vorrichtung zur Erwärmung der Flakes, wobei dies vorzugsweise eine Heizschnecke oder ein Vibrationswendelförderer ist. Die Aufgabe der Heizvorrichtung ist es, die Flakes auf Prozesstemperatur zu erwärmen, bevor sie in den Behandlungsreaktor kommen. Die Wärmeübertragung in einem Wärmetauscher bzw. in der Heizvorrichtung kann viel effizienter gestaltet werden als die Wärmeübertragung bei der Erwärmung der Flakes im Reaktor selbst.

Nach einer besonders bevorzugten Weiterbildung weist der Dekontaminationsreaktor eine konische Form auf, die sich in Richtung der Schwerkraft aufweitet. Diese Reaktorgestaltung hat den Vorteil, dass die erwärmten Flakes, die zur Brückenbildung neigen, an dieser gehindert werden. Weiterhin ist es möglich, dass der Reaktor eine Heizeinrichtung aufweist, die die Flakes zwar nicht aufheizt, dennoch aber auf Temperatur hält und somit Wärmeverluste ausgleicht.

Gemäß einer Weiterbildung sind mindestens zwei Dekontaminationsreaktoren vorgesehen, die vorzugsweise eine Gasdurchströmungseinrichtung aufweisen, um die Kontaminanten aus dem Reaktor abführen zu können. Es kann auch eine Gasdurchströmungseinrichtung für beide Behälter vorgesehen sein. Durch die Anordnung zweier Reaktoren können zum Beispiel verschiedene Flakes in verschiedenen Reaktoren behandelt werden. Dies kann zum Beispiel dann nötig werden, wenn sowohl dünnere "Rumpfflakes" bzw. Wandflakes als auch dickere "Halsflakes" von Getränkeflaschen verarbeitet werden sollen. Aufgrund der unterschiedlichen charakteristischen Länge der Flakes werden diese dann in zwei unterschiedliche Reaktoren mit unterschiedlichen Prozessparametern gegeben. Zwischen dem Dekontaminationsreaktor und dem SSP-Reaktor ist eine weitere Heizvorrichtung vorgesehen.

Während des Betriebes des Dekontaminationsreaktors ist die Füllhöhe und die Verweildauer der Flakes vorzugsweise nahezu konstant. Außerdem läuft der Betrieb des Reaktors vorzugsweise kontinuierlich ab, sodass immer genauso viele Flakes zugeführt werden, wie nach der Dekontamination abgeführt werden.

Auch der SSP-Reaktor weist vorzugsweise eine Gasdurchströmungseinrichtung auf, die Kontaminanten abführen kann. Da die Temperatur im SSP-Reaktor vorzugsweise zwischen 170° C und 210° C liegt, handelt es sich bei der Gasdurchströmungseinrichtung um eine Inertgasversorgung, vorzugsweise mit Stickstoff oder Kohlendioxid, da sonst die Gefahr besteht, dass Luftbestandteile bei diesen hohen Temperaturen einen Oxidationsprozess einleiten, der u.a. zu einer unerwünschten Gelbfärbung des Produkts führen kann.

In einer bevorzugten Ausführungsform weist der SSP-Reaktor eine Pumpe zur Erzeugung des Vakuums und eine Pumpe zur Abfuhr der Kontaminanten auf. Gemäß einer besonders bevorzugten Weiterbildung ist nur eine Pumpe vorhanden, die sowohl die Erzeugung des Vakuums als auch die Abfuhr der Kontaminanten im Gasstrom vornimmt.

Dem SSP-Reaktor nachgeschaltet ist eine Kühleinheit, die die Temperatur der Flakes von der des SSP-Prozesses auf eine Temperatur unterhalb des Glasübergangspunktes absenkt.

Gemäß einer besonders bevorzugten Ausführungsform stehen zur SSP-Behandlung mindestens zwei Reaktoren, vorzugsweise 3 bis 7 Einzelreaktoren zur Verfügung um einen quasi kontinuierlichen Prozess zu erzeugen. Durch die Anordnung mehrerer Reaktoren ist es möglich, den batchweise (absatzweise) arbeitenden Prozess der Einstellung des IV-Wertes durch verschiedene Reaktoren so durchzuführen, dass die der SSP-Reaktoreinheit nachgeordnete Kühleinrichtung kontinuierlich mit heißen Flakes aus den einzelnen Reaktoren der Reaktoreinheit versorgt wird.

Die SSP-Reaktoreinheit weist vorzugsweise einen Flakeverteiler auf, der die verschiedenen Einzelreaktoren nacheinander beschickt. Die Einzelreaktoren der SSP-Reaktoreinheit sind vorzugsweise in einer im Wesentlichen kreisförmigen Struktur angeordnet, um möglichst kompakt zu bauen.

Trotz der Tatsache, dass der SSP-Prozess batchweise abläuft, kann durch diese mehrteilige Reaktoranordnung ein quasikontinuierlicher Prozess erzeugt werden. Auf diese Weise können die Vorteile eines kontinuierlich ablaufenden Gesamtprozesses (z.B. Heiz- und Kühleinrichtungen können kontinuierlich betrieben werden) mit den Vorteilen des batchweise ablaufenden SSP-Prozesses (z.B. IV-Wert kann aufgrund der chargenweisen Behandlung genauer eingestellt werden) verknüpft werden.

Nach einer weiteren Ausführungsform sind sowohl mindestens zwei Dekontaminationsreaktoren als auch mindestens zwei SSP-Reaktoren vorhanden.

Die Behandlungszeiten im Dekontaminationsreaktor und im SSP-Reaktor hängen von dem anfänglichen IV-Wert und von der charakteristischen Länge bzw. von der Partikelgröße der Flakes ab. Sie liegen im Bereich von 20 min. bis 5 Stunden, vorzugsweise von 45 min. bis 2 Stunden, besonders bevorzugt jedoch von 1 bis 1,5 Stunden.

Eine Ausführungsform der Vorrichtung bzw. des Verfahrens wird anhand der Zeichnungen näher erläutert. Dabei zeigt:
- Fig.1: eine schematische Darstellung eines Verfahrensfließbildes,
- Fig. 2: eine schematische Darstellung der Draufsicht auf eine SSP-Reaktoreinheit.

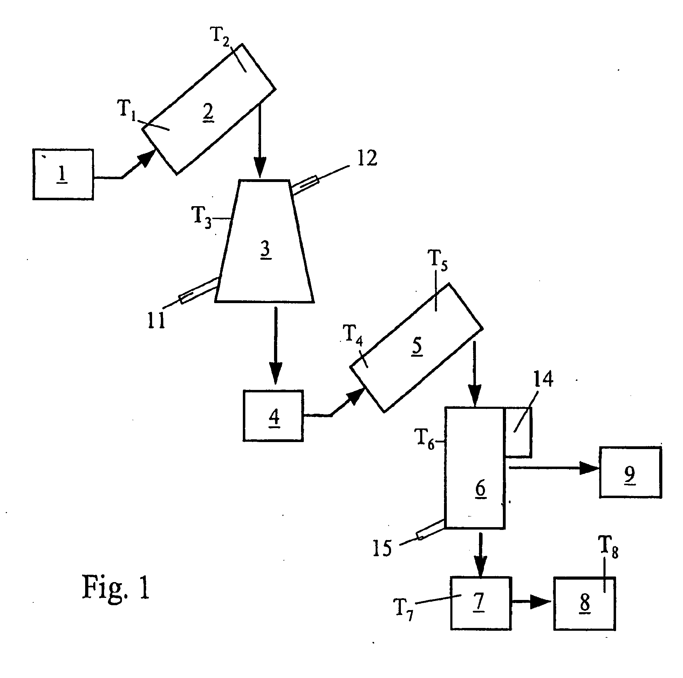

In Figur 1 ist eine Flakedosierungseinrichtung 1 gezeigt, durch die die Flakes in vorbestimmter Weise in die Dekontaminationsheizschnecke 2 gefördert werden. Bei der Zugabe in die Flakedosierungseinrichtung 1 sind die Flakes gewaschen und ihre Restfeuchtigkeit beträgt weniger als 3%. Zu Beginn des Transports der Flakes durch die Dekontaminationsheizschnecke 2 weisen sie Raumtemperatur T1 auf. (Es ist aber auch möglich, dass die Flakes von den vorherigen Prozessen noch eine Restwärme besitzen.) Während des Transports durch die mit Thermalöl betriebene Dekontaminationsheizschnecke 2 steigt die Temperatur von Raumtemperatur T1 auf Prozesstemperatur T2, wobei die Prozesstemperatur hier 150° C beträgt. Dabei kristallisieren die Flakes soweit, dass sie sich problemlos fördern lassen und nicht kleben.

Nach der Erwärmung der Flakes auf Prozesstemperatur werden diese in den Dekontaminationsreaktor 3 gegeben, der eine konische Form aufweist, die sich in Richtung der Schwerkraft aufweitet. Dort beginnt sofort die Dekontamination der Flakes. Ist die Füllhöhe im Dekontaminationsreaktor 3 erreicht, so werden die Flakes mit dem gleichen Durchsatz über die Flakedosierungseinrichtung 4 an die SSP-Heizschnecke 5 weitergegeben, wie sie von der Dekontaminationsheizschnecke 2 in den Dekontaminationsreaktor 3 kommen. Dadurch bleibt der Füllstand bei dieser kontinuierlichen Betriebsweise immer auf optimalem Niveau. Der Prozess wird dabei so eingestellt, dass eine Unterschreitung der Mindestverweildauer der Flakes im Dekontaminationsreaktor 3 nicht möglich ist.

Im Dekontaminationsreaktor 3 herrscht eine Temperatur T3 von 150° C vor. Die Kristallisation bzw. Dekontamination der Flakes wird in Abhängigkeit der beiden Faktoren Zeit und Temperatur durchgeführt. Ein unterstützendes Merkmal dieses Prozesses ist die Luftzufuhr, durch welche die aus den Flakes ausgetriebenen Kontaminanten abgeführt werden. Die Luft wird auf die Prozesstemperatur T3 vorgeheizt und über die Luftzufuhr 11 in den Dekontaminationsreaktor 3 eingeleitet, strömt durch die sich in dem Dekontaminationsreaktor 3 befindlichen Flakes und wird über die Luftabfuhr 12 ausgeleitet. Die Strömungsgeschwindigkeit der Luft darf einen Mindestwert nicht unterschreiten und wird dabei so eingestellt, dass die Flakes nicht aufgewirbelt werden. Der Luftstrom dient lediglich dazu, die aus den Flakes ausgetriebenen Kontaminanten leichter und schneller abzuführen. Die Luft weist im Wesentlichen eine Temperatur im Bereich der Prozesstemperatur auf. Im Dekontaminationsreaktor 3 erfolgt also keine Erwärmung der Flakes, sondern lediglich eine Temperaturstabilisierung auf Prozesstemperatur.

Da der Übergang der Flakes vom Dekontaminationsreaktor 3 in die Flakedosierungseinrichtung 4 und in die SSP-Heizschnecke 5 optimal isoliert ist und somit kein bzw. ein möglicht geringer Wärmeverlust entsteht, weisen die Flakes am Beginn der SSP-Heizschnecke 5, die die Flakes in den SSP-Reaktor 6 befördert, eine Temperatur T4 von 150° C auf. In der SSP-Heizschnecke 5, die mit Thermalöl betrieben wird, wird eine Erwärmung der Flakes von 150° C (T4) auf 190° C (T5) vorgenommen.

Um eine optimale Prozesssteuerung bezüglich des IV-Wertes der Flakes vornehmen zu können, wird die intrinsische Viskosität der Flakes in einer IV-Messvorrichtung 14, die sich am SSP-Reaktor befindet, nach bzw. während des Befüllvorgangs automatisch durchgeführt. Anhand dieses Messwertes wird die Verweildauer der Flakes im SSP-Reaktor 6 bestimmt.

Um die weitere Entfernung von Kontaminanten und die Erhöhung der inneren Viskosität zu erleichtern, wird am SSP-Reaktor 6, der eine im Wesentlichen zylinderförmige Form aufweist, ein Vakuum mit einem Gasdruck von 1 Millibar mittels einer Vakuumpumpe 9 angelegt. Zur besseren Abfuhr der Kontaminanten wird über eine Inertgaszufuhr 15 Stickstoff bzw. Kohlendioxid zugeführt, der die Flakes derart umströmt, dass sie nicht verwirbelt werden. Das mit den Kontaminanten angereicherte Inertgas wird über die Vakuumpumpe 9 abgesaugt. Im SSP-Reaktor 6 herrscht eine Temperatur T6 von 190° C vor.

Nach der Hälfte der ursprünglich berechneten Verweildauer der Flakes im SSP-Reaktor 6 wird durch die IV-Messvorrichtung 14 erneut eine Probe der Flakes bezüglich des Wertes der intrinsischen Viskosität analysiert. Aufgrund der Entwicklung des IV-Wertes der Flakes kann die restliche Verweildauer im SSP-Reaktor 6 kontrolliert bzw. korrigiert werden. Aufgrund der zweimaligen Messung des IV-Wertes während der SSP-Behandlung kann ein optimales Prozessergebnis bezüglich der intrinsischen Viskosität erzielt werden.

Nach der Behandlung im SSP-Reaktor 6 werden die Flakes in eine Flakedosiereinrichtung 7 gegeben, von wo aus sie dosiert dem Kühlreaktor 8 zugeführt werden können. In der Flakedosiereinrichtung 7 weisen die Flakes eine Temperatur T7 von ca. 190° C, also derjenigen Temperatur des SSP-Prozesses auf. Die Abkühlung der Flakes im Kühlreaktor 8 erfolgt mittels Umgebungsluft auf eine Temperatur T8 von ca. 70° C. Die Kühlung hat so schnell zu erfolgen, dass trotz der Feuchtigkeit die in der Umgebungsluft enthalten ist, keine Hydrolyse der Flakes stattfindet.

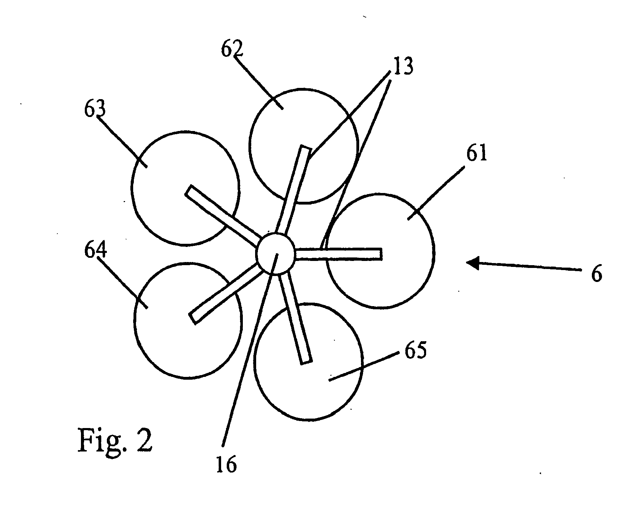

Figur 2 zeigt eine besondere Ausprägung des SSP-Reaktors 6. Dabei sind fünf, im Wesentlichen zylinderförmige SSP-Einzelreaktoren 61, 62, 63, 64 und 65 in einer kreisförmigen Struktur zu einem gesamten SSP-Reaktor 6 angeordnet. Nach der Erwärmung der Flakes von T4 auf T5 (150° C auf 190° C) in der SSP-Heizschnecke 5 werden sie über einen Verteilertrichter 16 und Verteilerkanäle 13 gesteuert in die einzelnen Reaktoren verteilt.

Die Flakes werden z. B. vom Verteilertrichter 16 über einen Verteilerkanal 13 solange in den SSP-Einzelreaktor 61 geleitet, bis dieser die Prozessfüllhöhe erreicht hat. Während im SSP-Einzelreaktor 61 der SSP-Prozess mit der Messung des IV-Wertes durch die IV-Messvorrichtung 14 beginnt, werden die von der SSP-Heizschnecke weiterhin kontinuierlich geförderten Flakes über den Verteilertrichter 16 und den Verteilerkanal 13 in den SSP-Einzelreaktor 62 geleitet, bis dieser die Produktionsfüllhöhe erreicht hat. Während nun der SSP-Prozess im SSP-Einzelreaktor 62 mit der Messung des IV-Wertes der Flakes beginnt, wird der SSP-Einzelreaktor 63 mit erhitzten Flakes befüllt. Auf diese Weise werden die SSP-Einzelreaktoren 61 bis 65 der Reihe nach mit Flakes befüllt.

Bis der SSP-Einzelreaktor 65 mit Flakes befüllt ist, ist der SSP-Prozess im SSP-Einzelreaktor 61 abgeschlossen und die Flakes sind über die Flakedosierungseinrichtung 7 in Richtung des Kühlreaktors 8 abtransportiert. Aus diesem Grund kann der SSP-Einzelreaktor 61 nach dem der SSP-Einzelreaktor 65 befüllt wurde auch wieder befüllt werden. Auf diese Weise kann der SSP-Prozess, der vorzugsweise batchweise durchgeführt wird, als quasi kontinuierlicher Prozess mit kontinuierlicher Flakezuführung von der SSP-Heizschnecke 5 und mit kontinuierlicher Flakeabführung von der Flakedosierungseinrichtung 7 in den Kühlreaktor 8 durchgeführt werden.

## Patentansprüche

1. Verfahren zur Aufbereitung von zu Flakes zerkleinertem kontaminierten R-PET, wobei die Aufbereitung mindestens die Schritte einer Dekontamination und einer "solid state polycondensation" (SSP-Behandlung) umfasst, **dadurch gekennzeichnet, dass** die Dekontamination in mindestens einem Dekontaminationsreaktor (3) und die SSP-Behandlung in mindestens einem SSP-Reaktor (6) durchgeführt wird,
wobei eine Erwärmung der Flakes auf Prozeßtemperatur des Dekontaminationsreaktors (3) in einer diesem vorgeschalteten Erwärmungsvorrichtung (2) erfolgt und dass eine weitere Erwärmung der Flakes auf eine höhere Temperatur als die Prozeßtemperatur im Dekontaminationsreaktor (6), nämlich die Prozeßtemperatur des SSP-Reaktors (6), in einer dem SSP-Reaktor (6) vorgeschalteten Erwärmungsvorrichtung (5) erfolgt.

2. Verfahren nach Anspruch 1, **dadurch gekennzeichnet, dass** in dem mindestens eine Reaktor (3, 6) kein mechanischer Energieeintrag in das aufzubereitende Material stattfindet.

3. Verfahren nach mindestens einem der Ansprüche 1 bis 2, **dadurch gekennzeichnet, dass** das aufzubereitende Material im Dekontaminationsreaktor (3) von heißem Gas umströmt wird.

4. Verfahren nach Anspruch 3, **dadurch gekennzeichnet, dass** das aufzubereitende Material von Luft umströmt wird.

5. Verfahren nach wenigstens einem der vorhergehenden Ansprüche, **dadurch gekennzeichnet, dass** die Dekontamination der Flakes im Dekontaminationsreaktor (3) kontinuierlich erfolgt.

6. Verfahren nach wenigstens einem der vorhergehenden Ansprüche, **dadurch gekennzeichnet, dass** im Dekontaminationsreaktor (3) eine Temperatur zwischen 20°C und 200°C vorherrscht.

7. Verfahren nach Anspruch 1, **dadurch gekennzeichnet, dass** die Erwärmung des aufzubereitenden Materials auf Prozesstemperatur vor dem Dekontaminationsreaktor (3) kontinuierlich erfolgt.

8. Verfahren nach Anspruch 7, **dadurch gekennzeichnet, dass** die Erwärmung in einer Heizschnecke oder in einem Vibrationswendelförderer erfolgt.

9. Verfahren nach Anspruch 8, **dadurch gekennzeichnet, dass** als Medium zur Übertragung der Wärme auf das aufzubereitende Material Thermalöl verwendet wird.

10. Verfahren nach wenigstens einem der vorhergehenden Ansprüche, **dadurch gekennzeichnet, dass** die SSP-Behandlung im SSP-Reaktor (6) batchweise durchgeführt wird.

11. Verfahren nach wenigstens einem der Ansprüche 1 bis 10, **dadurch gekennzeichnet, dass** im SSP-Reaktor (6) Unterdruckbedingungen vorherrschen.

12. Verfahren nach Anspruch 11, **dadurch gekennzeichnet, dass** im SSP-Reaktor (6) ein Vakuum mit einem Gasdruck von höchstens 100 mbar vorherrscht.

13. Verfahren nach mindestens einem der vorhergehenden Ansprüche, **dadurch gekennzeichnet, dass** die Prozesstemperatur im SSP-Reaktor (6) höher ist, als die im Dekontaminationsreaktor (3).

14. Verfahren nach Anspruch 13, **dadurch gekennzeichnet, dass** die Prozesstemperatur vorzugsweise zwischen 150°C und 250°C liegt.

15. Verfahren nach Anspruch 13, **dadurch gekennzeichnet, dass** die Temperaturerhöhung des aufzubereitenden Materials vor dem Eintritt in den SSP-Reaktor (6) kontinuierlich erfolgt.

16. Verfahren nach Anspruch 15, **dadurch gekennzeichnet, dass** die Temperaturerhöhung in einer Heizschnecke (5) oder in einem Vibrationswendelförderer erfolgt.

17. Verfahren nach Anspruch 15, **dadurch gekennzeichnet, dass** als Medium zur Übertragung der Wärme auf das aufzubereitende Material Thermalöl verwendet wird.

18. Verfahren nach wenigstens einem der Ansprüche 13 bis 17, **dadurch gekennzeichnet, dass** der SSP-Prozess unter Schutzgasatmosphäre durchgeführt wird.

19. Verfahren nach wenigstens einem der Ansprüche 13 bis 17, **dadurch gekennzeichnet, dass** ein Gasstrom durch das aufzubereitende Material geführt wird, der Abbauprodukte, Kontaminanten sowie Feinstpartikel aus dem SSP-Reaktor (6) abführt.

20. Verfahren nach wenigstens einem der vorhergehenden Ansprüche, **dadurch gekennzeichnet, dass** der IV-Wert des aufzubereitenden Materials vor bzw. während des Aufbereitungsprozesses mindestens ein Mal bestimmt wird.

21. Verfahren nach Anspruch 20, **dadurch gekennzeichnet, dass** das Ergebnis der IV-Wert-Messung zumindest Teile des Prozessablaufes bestimmt.

22. Verfahren nach wenigstens einem der Ansprüche 1 bis 21, **dadurch gekennzeichnet, dass** eine Messung des IV-Wertes vor dem Eintritt des aufzubereitenden Materials in den SSP-Reaktor (6) durchgeführt wird und anhand dessen Ergebnisses eine Verweildauer des Materials im Reaktor bestimmt wird.

23. Verfahren nach wenigstens einem der Ansprüche 21 bis 22, **dadurch gekennzeichnet, dass** eine weitere Messung des IV-Wertes nach im Wesentlichen der Hälfte der bestimmten Verweildauer des aufzubereitenden Materials im SSP-Reaktor (6) durchgeführt wird und anhand dessen Ergebnisses die vorher bestimmte Verweildauer im SSP-Reaktor (6) kontrolliert und gegebenenfalls korrigiert wird.

24. Verfahren nach wenigstens einem der vorangegangenen Ansprüche 1 bis 21, **dadurch gekennzeichnet, dass** das aufzubereitende Material nach der Verweildauer im SSP-Reaktor (6) auf eine Temperatur unterhalb des Glasübergangspunktes, vorzugsweise auf eine Temperatur zwischen 50°C und 100°C abgekühlt wird.

25. Verfahren nach Anspruch 24, **dadurch gekennzeichnet, dass** die Abkühlung in einer Kühlschnecke (8), einem Vibrationswendelförderer (8) oder in einem Wirbelschichtreaktor (8) vorgenommen wird.

26. Verfahren nach wenigstens einem der Ansprüche 24 bis 25, **dadurch gekennzeichnet, dass** die Abkühlung durch Umgebungsluft erfolgt.

## Claims

1. Process for cleaning contaminated R-PET that has been crushed and/or ground into flakes, whereby the cleaning includes at least a decontamination stage and a solid state polycondensation (SSP) stage, **characterized in that** the decontamination is carried out in at least one decontamination reactor (3) and the solid state polycondensation (SSP) is carried out in at least one SSP reactor (6), whereby heating of the flakes to the process temperature of the decontamination reactor (3) occurs in an equipment for heating (2) provided before the decontamination reactor, and that further heating of the flakes to a temperature higher than the process temperature of the decontamination reactor (3), namely to the process temperature of the SSP reactor (6), occurs in an equipment for heating (5) provided before the SSP reactor (6).

2. Process in accordance with claim 1, **characterized in that** there is no input of energy into the material to be cleaned, in the at least one reactor (3, 6).

3. Process in accordance with at least one of claims 1 to 2, **characterized in that** hot gas flows round the material to be cleaned in the decontamination reactor (3).

4. Process in accordance with claim 3, **characterized in that** air flows round the material to be washed.

5. Process in accordance with at least one of the preceding claims, **characterized in that** decontamination of the flakes occurs continuously in the decontamination reactor (3).

6. Process in accordance with at least one of the preceding claims, **characterized in that** the temperature in the decontamination reactor (3) is between 20° and 200°C.

7. Process in accordance with claim 1, **characterized by** continuous heating of the material to be cleaned at the process temperature, before the decontamination reactor (3).

8. Process in accordance with claim 7, **characterized in that** the heating occurs in a heating-screw conveyor or in a vibrating spiral conveyor.

9. Process in accordance with claim 8, **characterized in that** thermal oil is used as a medium for transferring heat to the material to be washed.

10. Process in accordance with one of the preceding claims, **characterized in that** the SSP treatment is carried out in batches in the SSP reactor (6).

11. Process in accordance with at least one of claims 1 to 10, **characterized in that** the pressures in the SSP reactor (6) are negative.

12. Process in accordance with claim 11, **characterized in that** there is a vacuum in the SSP reactor (6) with a gas pressure of at most 100 mbar.

13. Process in accordance with at least one of the preceding claims, **characterized in that** the process temperature in the SSP reactor (6) is higher than the temperature in the decontamination reactor (3).

14. Process in accordance with claim 13, **characterized in that** the process temperature preferably lies between 150° and 250°C.

15. Process in accordance with claim 13, **characterized in that** the temperature of the material to be washed is continuously increased before entry into the SSP reactor (6).

16. Process in accordance with claim 15, **characterized in that** the increasing of the temperature occurs continuously in a heating-screw conveyor (5) or in a vibrating spiral conveyor.

17. Process in accordance with claim 15, **characterized in that** thermal oil is used to transfer the heat to the material to be cleaned.

18. Process in accordance with one of claims 13 to 17, **characterized in that** the SSP process occurs in an inert gas.

19. Process in accordance with one of claims 13 to 17, **characterized in that** derivatives, contaminants and micro-particles are removed from the SSP reactor by a gas flow that is directed through the material to be washed.

20. Process in accordance with at least one of the preceding claims, **characterized in that** the intrinsic viscosity of the material to be cleaned is measured at least once before or during the cleaning process.

21. Process in accordance with claim 20, **characterized in that** the intrinsic viscosity measurement determines at least parts of the process.

22. Process in accordance with at least one of claims 1 to 21; **characterized in that** the intrinsic viscosity (IV) is measured before entry of the material to be washed into the SSP reactor (6), and **in that** the time-period during which the material remains in the reactor is determined in connection with the result.

23. Process in accordance with at least one of claims 21 and 22, **characterized in that** a further measurement of the IV is occurs after substantially half the specified time-period in which the material is to be washed in the SSP reactor (6), and **in that** the previously determined period of time in the SSP reactor (6) is verified, and where applicable corrected, on the basis of this measurement.

24. Process in accordance with at least one of claims 1 to 21, **characterized in that**, following the time-period passed in the SSP reactor (6), the material to be cleaned is cooled to a temperature below the glass transition point, and preferably to a temperature between 50°C and 100°C.

25. Process in accordance with claim 24, **characterized in that** cooling occurs in a cooling spiral (8), a vibrating spiral conveyor (8) or in a layer turbulence reactor (8).

26. Process in accordance with at least one of claims 24 to 25, **characterized in that** the cooling occurs by means of ambient air.

## Revendications

1. Procédé pour préparer des paillettes de matières plastiques contaminées, selon lequel la préparation comporte au moins les étapes comprenant une décontamination et une polycondensation à l'état solide (opération SSP)
**caractérisé en ce que**
la décontamination se fait dans au moins un réacteur de décontamination (3) et le traitement SSP se fait dans au moins un réacteur SSP (6), moyennant quoi un chauffage des paillettes jusqu'à la température de procédé du réacteur de décontamination (3) a lieu dans un dispositif de chauffage (2) prévu en amont du réacteur de décontamination (3), et un chauffage supplémentaire des paillettes jusqu'à une température plus élevée que la température de procédé du réacteur de décontamination (3), à savoir la température de procédé du réacteur SSP (6), a lieu dans un dispositif de chauffage (5) prévu en amont du réacteur SSP (6).

2. Procédé selon la revendication 1,
**caractérisé en ce que**
dans au moins un réacteur (3, 6) il n'y a pas d'apport d'énergie mécanique à la matière à traiter.

3. Procédé selon au moins l'une des revendications 1 à 2,
**caractérisé en ce que**
la matière à traiter dans le réacteur de décontamination (3) est balayée par un gaz chaud.

4. Procédé selon la revendication 3,
**caractérisé en ce que**
la matière à traiter est balayée par de l'air.

5. Procédé selon au moins l'une des revendications précédentes,
**caractérisé en ce que**
la décontamination des paillettes se fait en continu dans le réacteur de décontamination.

6. Procédé selon au moins l'une des revendications précédentes,
**caractérisé en ce qu'**
on a une température comprise entre 20°C et 200°C dans le réacteur de décontamination (3).

7. Procédé selon la revendication 1,
**caractérisé en ce que**
l'élévation de température de la matière à traiter jusqu'à la température du procédé se fait en continu en amont du réacteur de décontamination (3).

8. Procédé selon la revendication 7,
**caractérisé en ce que**
l'élévation de température se fait dans une vis de chauffage ou dans un convoyeur vibrant à hélice.

9. Procédé selon la revendication 8,
**caractérisé en ce que**
comme milieu pour transférer la chaleur à la matière à travailler, on utilise de l'huile thermique.

10. Procédé selon au moins l'une des revendications précédentes,
**caractérisé en ce que**
le traitement SSP se fait par lots dans le réacteur SSP (6).

11. Procédé selon au moins l'une des revendications 1 à 10,
**caractérisé en ce que**
des conditions de dépression règnent dans le réacteur SSP (6).

12. Procédé selon la revendication 11,
**caractérisé en ce qu'**
un vide à une pression de gaz au plus égale à 100 mbar règne dans le réacteur SSP (6).

13. Procédé selon au moins l'une des revendications précédentes,
**caractérisé en ce que**
la température de procédé dans le réacteur SSP (6) est supérieure à celle dans le réacteur de décontamination (3).

14. Procédé selon la revendication 13,
**caractérisé en ce que**
la température de procédé est comprise de préférence entre 150°C et 250°C.

15. Procédé selon la revendication 13,
**caractérisé en ce que**
l'élévation de température de la matière à traiter se fait en continu avant son entrée dans le réacteur SSP (6).

16. Procédé selon la revendication 15,
**caractérisé en ce que**
l'élévation de température se fait dans une vis chauffante (5) ou dans un convoyeur vibrant à hélice.

17. Procédé selon la revendication 15,
**caractérisé en ce que**
le milieu de transfert de chaleur à la matière à préparer est de l'huile thermique.

18. Procédé selon au moins l'une des revendications 13 à 17,
**caractérisé en ce que**
le procédé SSP est effectué sous une atmosphère de gaz protecteur.

19. Procédé selon au moins l'une des revendications 13 à 17,
**caractérisé en ce qu'**
on fait passer une veine de gaz à travers la matière à traiter, cette veine de gaz évacuant les produits de décomposition, les produits de contamination ainsi que les particules les plus fines hors du réacteur SSP (6).

20. Procédé selon au moins l'une des revendications précédentes,
**caractérisé en ce qu'**
on détermine au moins une fois la valeur IV de la matière à traiter avant ou pendant le procédé de traitement.

21. Procédé selon la revendication 20,
**caractérisé en ce que**
le résultat de la mesure de la valeur IV détermine au moins des parties de l'exécution du procédé.

22. Procédé selon au moins l'une des revendications 1 à 21,
**caractérisé en ce qu'**
on effectue la mesure de la valeur IV avant que la matière à traiter entre dans le réacteur SSP (6) et à l'aide de ce résultat de mesure, on définit le temps de séjour de la matière dans le réacteur.

23. Procédé selon au moins l'une des revendications 21 à 22,
**caractérisé en ce qu'**
on effectue une autre mesure de la valeur IV après pratiquement la moitié du temps de séjour déterminé de la matière à traiter dans le réacteur SSP (6) et à l'aide de ce résultat, on contrôle et, le cas échéant, on corrige le temps de séjour préalablement déterminé dans le réacteur SSP (6).

24. Procédé selon au moins l'une des revendications précédentes 1 à 21,
**caractérisé en ce qu'**
après le temps de séjour dans le réacteur SSP (6) on refroidit la matière à traiter à une température inférieure à la température de transition vitreuse, de préférence à une température comprise entre 50°C et 100°C.

25. Procédé selon la revendication 24,
**caractérisé en ce qu'**
on effectue le refroidissement dans une vis de refroidissement (8), dans un convoyeur vibrant à hélice (8) ou dans un réacteur à lit fluidisé (8).

26. Procédé selon au moins l'une des revendications 24 à 25,
**caractérisé en ce qu'**
on effectue le refroidissement avec l'air ambiant.
